(19)

Europäisches
Patentamt
European
Patent Office
Office européen
des brevets

(11) **EP 2 387 996 A1**

(12) **EUROPEAN PATENT APPLICATION**

(43) Date of publication:
    **23.11.2011 Bulletin 2011/47**

(51) Int Cl.:
    **A61K 31/343** (2006.01)   **A61P 9/00** (2006.01)

(21) Application number: **10305514.1**

(22) Date of filing: **17.05.2010**

(84) Designated Contracting States:
    **AL AT BE BG CH CY CZ DE DK EE ES FI FR GB
    GR HR HU IE IS IT LI LT LU LV MC MK MT NL NO
    PL PT RO SE SI SK SM TR**
    Designated Extension States:
    **BA ME RS**

(71) Applicant: **SANOFI
    75013 Paris (FR)**

(72) Inventor: **The designation of the inventor has not
    yet been filed**

(74) Representative: **Bouron, Estelle
    Sanofi-Aventis
    Département Brevets
    174, avenue de France
    75013 Paris (FR)**

(54) **Use of dronedarone for the preparation of a medicament for the prevention of cardiovascular events in patients with permanent atrial fibrillation**

(57) Use of dronedarone for the preparation of a medicament for the prevention of cardiovascular events notably stroke, acute coronary syndrome or cardiovascular death in patients with permanent atrial fibrillation.

EP 2 387 996 A1

**Description**

The instant invention relates to the use of dronedarone for the preparation of a medicament for the prevention of cardiovascular events notably stroke, acute coronary syndrome or cardiovascular death in patients with permanent atrial fibrillation.

2-n-butyl-3-[4-(3-di-n-butylaminopropoxy)benzoyl]-5-methylsulfonamido-benzofuranne or dronedarone and its pharmaceutically acceptable salts are described in European patent EP 0 471 609 B1.

Dronedarone is a multi-channel blocker that affects calcium, potassium and sodium channels and has anti-adrenergic properties.

Dronedarone is an anti-arrhythmic agent for the treatment of patients with a history of atrial fibrillation or atrial flutter.

Atrial fibrillation (AF) affects about 2.3 million people in North America and 4.5 million people in the European Union and is emerging as a growing public health concern because of the aging of the population

AF is a condition in which the upper chambers of the heart beat in an uncoordinated and disorganized fashion, resulting in a very irregular and fast rhythm (i.e., an irregularly, irregular heartbeat). When blood is not completely pumped out of the heart's chambers, it can pool and clot. If a blood clot forms in the atria, exits the heart and blocks an artery in the brain, a stroke results. Consequently, about 15 percent of strokes result from AF. But stroke can also complicate other conditions like for example hypertension. Also hemorrhagic strokes can be a complication of treatment with an anticoagulant prescribed to prevent the formation of thrombi in particular in patients with AF.

AF is increasingly frequent with advancing age and is often caused by age-related changes in the heart, physical or psychological stress, agents that stimulate the heart, such as caffeine, or as a result of cardiovascular disease. The number is expected to double in the next 20 years. Without appropriate management, AF can lead to serious complications, such as stroke and congestive heart failure.

As most of the studies did not assess the complications associated with atrial fibrillation such as stroke, so the effect of antiarrhythmic drugs on these endpoints is unknown (Cochrane Collaboration, The Cochrane Library, 2008, 2).

In addition, two large studies including antiarrhythmic drugs in AF patients, AFFIRM (D.G. Wyse and al., The New England Journal of Medecine, 2002, vol. 347, p.1825-1833) and AF-CHF (D. Roy and al., The New England Journal of Medecine, 2008, vol. 358, p.2667-2677), did not show a significant difference in stroke rates between the rate and rhythm groups (the recommended antiarrhythmic drug in the rhythm group was mainly amiodarone).

Thromboembolic events including strokes are major complications in patients with atrial fibrillation. The etiology of these thromboembolic events are not fully understood. According to the main hypothesis atrial fibrillation leads to blood stasis in the atria, which promotes the formation of blood clots and thereby causes thromboembolic events like stroke if the blood clots reach the systemic circulation. Therefore it was thought that prevention of atrial fibrillation or anticoagulation would prevent thromboembolic events and strokes. Numerous clinical studies have confirmed that proper anticoagulation can prevent thromboembolic events including strokes (Fuster et al.). But, all randomized clinical trials using anti-arrhythmic drugs did not show a reduction in the incidence of stroke, despite effectively maintaining sinus rhythm in the rhythm control or treatment group.

For example, in the AFFIRM trial, Wyse et al. compared a rhythm control (63% amiodarone and 41 % sotalol being the most commonly used anti-arrhythmic drugs) to a rate control strategy. As shown in Table 3 of the article by Wyse et al the incidence of stroke or TIA was similar in the rhythm control group (80/2033) compared to the rate control group (77/2027), despite a higher number of patients in the rhythm control group (63%) being in sinus rhythm after 5 years compared to the rate control group (35%).

In the STAF trial, Carlsson el al. compared a rhythm control (42% amiodarone) to a rate control strategy. As shown in Table 2 of the article by Carlsson et al the incidence of stroke or TIA was numerically higher in the rhythm control group (5/100) compared to the rate control group (1/100), despite a highly significant 29% absolute increase in patients with sinus rhythm at the end of the study in the rhythm control group compared to the rate control group.

In the HOT CAFÉ, Opolski et al compared a rate and a rhythm control strategy. As shown in table 2 of the article of Opolski et al 3/104 patients suffered from a stroke during the follow-up in the rhythm control group compared to 0/101 in the rate control group.

In the J-RHYTHM trial, Ogawa et al. compared a rhythm control (85% of patients were on class I anti-arrhythmic drugs) to a rate control strategy. As shown in Table 3 of the article by Ogawa et al the incidence of symptomatic stroke was similar in the rhythm control group (9/419) compared to the rate control group (11/404), despite a highly significant 29% absolute increase in patients with sinus rhythm at 3 years in the rhythm control group compared to the rate control group.

In the SAFE-T trial, Singh et al compared amiodarone, sotalol and placebo in the treatment of patients with persistent atrial fibrillation. Amiodarone and sotalol were both significantly more effective than placebo in increasing the time to a recurrence of atrial fibrillation (a widely used measure for rhythm control) (P<0.001). Amiodarone was six times as effective as sotalol in the intention-to-treat analysis (P<0.001) and four times as effective in the analysis according to the treatment actually received (P<0.001). Despite this effective rhythm control the number of strokes per 100 patient-years of follow-up were similar in all groups for amiodarone: 2.06 major, sotalol: 2.71, and placebo: 1.91 with the lowest rate observed in the placebo group, which had the highest rate of recurrence of atrial fibrillation (calculated from bottom of last paragraph on page 1866)).

Therefore, administering a drug for preventing atrial fibrillation cannot be considered as implying a prevention of stroke, according to the current knowledge in the Art. Unexpectedly, dronedarone has demonstrated, in the ATHENA trial (Hohnloser et al.), its ability to reduce the incidence of stroke. The effect now seen with dronedarone is not based upon rhythm control alone but on the unique combination of properties of dronedarone, which include but are not limited to: effective rhythm control, heart rate lowering effects, blood pressure lowering effects, direct effects on the endothelial function and others.

The Inventors have now clinically proven that dronedarone reduces in patients with permanent atrial fibrillation the occurrence of cardiovascular events such as stroke, acute coronary syndrome or cardiovascular death while this was not demonstrated for other antiarrhythmic compounds.

The subject of the instant invention is the use of dronedarone or one of its pharmaceutically acceptable salts for the preparation of a medicament for the prevention of cardiovascular events in patients with permanent atrial fibrillation.

Patients in "permanent atrial fibrillation or flutter" are patients that have all scheduled ECGs in this rhythm throughout the period the dronedarone or a pharmaceutically acceptable salt thereof is administered.

Cardiovascular events may be defined as stroke, acute coronary syndrome or cardiovascular death.

According to one embodiment, the subject of the instant invention is also the use of dronedarone or one of its pharmaceutically acceptable salts for the preparation of a medicament for the prevention of stroke, acute coronary syndrome or cardiovascular death more particularly the prevention of about 20% of stroke, acute coronary syndrome or cardiovascular death in patients with permanent atrial fibrillation.

According to one embodiment, the subject of the instant invention is also the use of dronedarone or one of its pharmaceutically acceptable salts for the preparation of a medicament for the prevention of stroke in patients with permanent atrial fibrillation.

According to one embodiment, the subject of the instant invention is also the use of dronedarone or one of its pharmaceutically acceptable salts for the preparation of a medicament for the prevention of acute coronary syndrome in patients with permanent atrial fibrillation.

According to one embodiment, the subject of the instant invention is also the use of dronedarone or one of its pharmaceutically acceptable salts for the preparation of a medicament for the prevention of cardiovascular death in patients with permanent atrial fibrillation.

Mention may be made that "use of dronedarone for the preparation of a medicament for use in the treatment/prevention of" may be understood as "dronedarone for the treatment/prevention of ".

In contrast to cerebral circulatory insufficiency, which is a chronic disease with slowly deteriorating cognitive function a stroke is an acutely or subacutely evolving neurological deficit of cerebrovascular cause defined by symptoms that persists beyond 24 hours due to a disturbance in the blood vessels of the brain or defined by imaging of an acute clinically relevant brain lesion in patients with rapidly vanishing symptoms.

This can be due to ischemia (lack of blood supply) caused by thrombosis or embolism, or due to a haemorrhage. (R.L. Sacco et al., Stroke, 2006; vol. 37 p.577-617)

Stroke can cause permanent neurological damage or death. It is the leading cause of adult disability in the United States and Europe.

Risk factors for stroke include advanced age, hypertension, previous stroke or transient ischemic attack (TIA), diabetes, high cholesterol, cigarette smoking, atrial fibrillation, etc. Hypertension is the most important modifiable risk factor of stroke.

The symptoms of a stroke are similar to those of a transient ischemic attack but last more than 24 hours.

The composite endpoint of stroke, acute coronary syndrome or cardiovascular death is a classical outcome measure in cardiovascular outcomes trials also called MACE (major adverse cardiovascular events) endpoint. The inclusion of this endpoint, shows the broader impact and relevance of the finding on stroke.

The data on ACS alone are connected to stroke, by the fact that both are ischemic events.

In terms of clinical study, the prevention of " stroke, acute coronary syndrome or cardiovascular death " constitute what are referred to as composite criteria or a combined endpoint.

The percentages above correspond to an average.

Among the pharmaceutically acceptable salts of dronedarone, mention may be made of the hydrochloride.

The treated patients may be patients with a history of atrial fibrillation or atrial flutter.

The expression « with a history of atrial fibrillation or atrial flutter » means a patient who has previously manifested at least one symptom of atrial fibrillation (AF) or atrial flutter (AFL) and who can be either in sinus rhythm or in atrial fibrillation or atrial flutter at the time of dronedarone administration.

It will also be specified that the expression " patients having a history of atrial fibrillation or atrial flutter", "patients with a history of or a current atrial fibrillation or flutter" or "patients with a recent history of or a current atrial fibrillation or flutter" or "patients with paroxysmal or persistent atrial fibrillation or flutter" or "patients with a history of, or a current paroxysmal or persistent atrial fibrillation or flutter" or "patients with a recent history of, or a current paroxysmal or persistent atrial fibrillation or flutter" or "patients with paroxysmal or intermittent atrial fibrillation or atrial flutter and a recent episode of atrial fibrillation or atrial flutter, who are in sinus rhythm or who will be cardioverted" or "patients with paroxysmal or persistent atrial fibrillation or atrial flutter and a recent episode of atrial fibrillation or atrial flutter, who are in sinus rhythm or who will be cardioverted" means a patient who, in the past, has presented one or more episodes of atrial fibrillation or flutter and/or who is suffering from atrial fibrillation or atrial flutter at the time the dronedarone or a pharmaceutically acceptable salt thereof is used. More particularly, this expression means patients with documentation of having been in both atrial fibrillation or flutter and sinus rhythm within the last 6 months preceding the start of treatment. Patients could be either in sinus rhythm, or in atrial fibrillation or flutter at the time the dronedarone or a pharmaceutically acceptable salt thereof is initiated.

It will also be specified that the terms "persistent" and intermittent" are equivalent.

In the instant invention, "atrial fibrillation" means atrial fibrillation and/or atrial flutter.

Among patients with permanent atrial fibrillation, mention may be made of patients who further have at least one of the following risk factors :

- age, notably equal to or above 70, or even above 75,
- hypertension,
- diabete,
- prior cerebrovascular accident or systemic embolism,
- left atrium diameter greater that or equal to 50 mm by echocardiography,
- left ventricular ejection fraction less than 40% by 2D-echocardiography.

Among patients with permanent atrial fibrillation, mention may also be made of patients having additional risk factors corresponding to at least one of the following diseases:

- hypertension,
- structural heart disease,
- tachycardia,
- coronary heart disease,
- non-rheumatic valvular heart disease,
- ischemic dilated cardiomyopathy,
- a history of ablation for AF/AFL for example catheter ablation or surgical ablation,
- supra-ventricular tachycardia other than AF/AFL,
- history of cardiac valve surgery,
- non-ischemic dilated cardiomyopathy,
- hypertrophic cardiomyopathy,
- rheumatic valvular heart disease,
- sustained ventricular tachycardia,
- congenital heart disease,
- a history of ablation for other reason than AF/AFL for example catheter ablation,
- ventricular fibrillation,

and/or at least a cardiac device chosen among:

- a pacemaker,
- an implanted cardioverter defibrillator.

Another object of the invention is a pharmaceutical composition which comprises, as active principle, dronedarone or one of its pharmaceutically acceptable salts. This pharmaceutical composition comprises an effective dose of at least one compound of formula (I) according to the invention, or an addition salt thereof with a pharmaceutically acceptable salt, or a hydrate or solvate thereof, and at least one pharmaceutically acceptable excipient. Said excipients are chosen according to the pharmaceutical form and the administration route desired, among usual excipients known to one of skill in the art.

In the pharmaceutical compositions according to the invention for the oral, sublingual, sub-cutaneous, intramuscular, intra-venous, topical, local, intratracheal, intranasal, transdermal or rectal administration, dronedarone or one of its salt, solvate or hydrate, can be administered as a unitary dosage form, in blend with usual pharmaceutical excipients, to animals and human beings for the prevention or for the treatment of pathological states mentioned above. The appropriate unitary dosage forms comprise the oral forms, such as tablets, hard or soft gelatin capsules, powders, granules and oral solutions or suspensions, the sublingual, buccal, intratracheal, intraocular, intranasal forms, the forms adapted to inhalation, topical, transdermal, sub-cutaneous, intramuscular or intra-venous delivery, the rectal forms and the implants. For the topical application, the compounds of the invention may be used as creams, gels, ointments or lotions.

For its use in therapeutics, dronedarone and its pharmaceutically acceptable salts are incorporated in pharmaceuticals compositions.

These pharmaceutical compositions comprise an effective dose of at least dronedarone or one of its pharmaceutically acceptable salts and at least one pharmaceutically acceptable excipient.

Said excipients are chosen according to the pharmaceutical form and the administration route desired, among usual excipients known of one of skill in the art.

In the pharmaceutical compositions for the oral, sublingual, sub-cutaneous, intramuscular, intra-venous, topical, local, intratracheal, intranasal, transdermal or rectal administration, dronedarone or one of its pharmaceutically acceptable salts, can be administered as a unitary dosage form, in blend with usual pharmaceutical excipients, to animals and human in diseases above mentioned.

The appropriate unitary dosage forms comprise the oral forms, such as tablets, hard or soft gelatin capsules, powders, granules and oral solutions or suspensions, the sublingual, buccal, intratracheal, intraocular, intranasal forms, by inhalation, the topical, transdermal, sub-cutaneous, intramuscular or intra-venous forms, the rectal forms and the implants. For the topical application, the compounds of the invention may be used as creams, gels, ointments or lotions.

As an example, a unitary dosage form for dronedarone or one of its pharmaceutically acceptable salts, in the form of a tablet, can comprise the following ingredients:

| Ingredients | mg |
| --- | --- |
| dronedarone hydrochloride (corresponding to 400 mg of base) | 426 |
| Methylhydroxypropylcellulose | 21,1 |
| Lactose monohydrate | 46,55 |
| Modified corn starch | 45,5 |
| Polyvinylpyrrolidone | 65 |
| Poloxamer 407 | 40 |
| Anhydrous colloidal silica | 2,6 |
| magnesium stearate | 3,25 |
|  | 650 |

| Ingredients | mg |
| --- | --- |
| dronedarone hydrochloride (corresponding to 400 mg of base) | 426 |
| microcristalline cellulose | 65 |
| Anhydrous colloidal silica | 2,6 |
| anhydrous lactose | 42,65 |
| Polyvinylpyrrolidone | 13 |
| Poloxamer 407 | 40 |
| Macrogol 6000 | 57,5 |
| magnesium stearate | 3,25 |
|  | 650 |

| Ingredients | mg |
|---|---|
| dronedarone hydrochloride (corresponding to 400 mg of base) | 426 |
| microcristalline cellulose | 26 |
| corn starch | 45,5 |
| Polyvinylpyrrolidone | 65 |
| Poloxamer 407 | 40 |
| Anhydrous colloidal silica | 3,25 |
| magnesium stearate | 3,25 |
| Lactose monohydrate | 41,65 |
|  | 650 |

| Ingredients | mg |
|---|---|
| dronedarone hydrochloride (corresponding to 400 mg of base) | 213 |
| microcrystalline cellulose | 13 |
| corn starch | 22,75 |
| Polyvinylpyrrolidone | 32,5 |
| Poloxamer 407 | 20 |
| Anhydrous colloidal silica | 1,3 |
| magnesium stearate | 1,625 |
| Lactose monohydrate | 20,825 |
|  | 650 |

Said pharmaceutical composition may be given once or twice a day with food.

The dose of dronedarone administered per day, orally, may reach 800 mg, taken in one or more intakes, for example one or two.

More specifically, the dose of dronedarone administered may be taken with food.

More specifically, the dose of dronedarone administered per day, orally, may reach 800 mg, taken in two intakes with a meal.

The dose of dronedarone administered per day, orally may be taken at a rate of twice a day with a meal for example with the morning and the evening meal.

More specifically, the two intakes may comprise same quantity of dronedarone.

In specific cases, higher or lower dosages may be appropriate; these dosages are comprised within the scope of the present invention. According to usual practice, the dosage suitable to each patient is determined by the physician according to the administration route, the weight, the disease, the body surface, the cardiac output and response of the patient.

The instant invention also relates to a method of prevention of stroke which comprises the administration to a patient of an effective dose of at least dronedarone or one of its pharmaceutically acceptable salts.

Efficacy of dronedarone and its pharmaceutically acceptable salts versus placebo for the prevention of stroke was provided via dronedarone hydrochloride during a prospective, multinational, double-blind, randomized, multi-center, placebo-controlled, parallel group trial.

## I. Selection of patients

Eligible patients have a history of atrial fibrillation or atrial flutter and/or may be in normal sinus rhythm or in atrial fibrillation or flutter at the time of recruitment.

Recruitment of patients was conducted taking into account the following inclusion criteria:

Inclusion criteria :

1) One of the following risk factors had to be present:

- age equal to or greater than 70 years,
- hypertension (taking antihypertensives of at least two different classes),
- diabetes,
- history of cerebral stroke (transient ischemic event or completed cerebral stroke) or of systemic embolism,
- left atrial diameter greater than or equal to 50 mm measured by echocardiography,
- left ventricular ejection fraction less than 40%, measured by two-dimensional echography;
  or
- age equal to or above 70, or even above 75, possibly combined with at least one of the risk factors below:

  o hypertension (taking antihypertensives of at least two different classes),
  o diabetes,
  o history of cerebral stroke (transient ischemic event or completed cerebral stroke) or of systemic embolism,
  o left atrial diameter greater than or equal to 50 mm measured by echocardiography,
  o left ventricular ejection fraction less than 40%, measured by two-dimensional echography;

2) availability of one electrocardiogram within the last six months, showing that the patients was or is in atrial fibrillation/flutter,

3) availability of one electrocardiogram within the last six months, showing that the patients was or is in sinus rhythm.

## II. Duration and treatment

Study drug treatment units (placebo or dronedarone hydrochloride corresponding to 400 mg of base) were such that each patient took one tablet in the morning during or shortly after breakfast and one tablet in the evening during or shortly after dinner.
The treatment duration depended on the time of recruitment of each patient in the trial and could be comprised from 12 months to 30 months.

## III. Results

Results were calculated using non-parametric Kaplan-Meier estimate.
Cox's proportional hazard model was used to estimate the hazard ratio also called relative risk.
Relative risk (RR) is the ratio between the risk of having a cardiovascular event for patients treated with dronedarone and the risk of having a cardiovascular event for patients treated with placebo.
Percentage of decrease of an event is calculated as follow:

$$x = 1 - RR.$$

Results relating to the prevention of stroke, Acute Coronary Syndrome (ACS) or cardiovascular death

From the 4628 patients included in the trial, 2301 were part of the group treated with dronedarone hydrochloride.
295 patients developed permanent atrial fibrillation/flutter in the group treated with placebo versus 178 patients in the group treated with dronedarone hydrochloride.
Patients in "permanent atrial fibrillation or flutter" are patients that have all scheduled ECGs in this rhythm throughout the period the dronedarone or a pharmaceutically acceptable salt thereof is administered.
24 events were reported in the placebo group versus 12 in the group treated with dronedarone hydrochloride.
Calculated relative risk was equal to 0.805, i.e. a decrease of the relative risk of stroke, Acute Coronary Syndrome (ACS) or cardiovascular death of 20%.

**Claims**

1. Use of dronedarone for the preparation of a medicament for the prevention of cardiovascular events in patients with permanent atrial fibrillation.

2. Use of dronedarone for the preparation of a medicament for the prevention of stroke, acute coronary syndrome or cardiovascular death in patients with permanent atrial fibrillation.

3. Use according to any one of claims 1 or 2 for the preparation of a medicament for the prevention of stroke.

4. Use according to any one of claims 1 or 2 for the preparation of a medicament for the prevention of acute coronary syndrome.

5. Use according to any one of claims 1 or 2 for the preparation of a medicament for the prevention of cardiovascular death.

6. Use according to any one of claims 1 to 5, wherein said prevention is provided to patients with permanent atrial fibrillation with a history of, or a current atrial fibrillation or atrial flutter.

7. Use according to any one of the preceding claims, **characterized in that** the patients with permanent atrial fibrillation have at least one of the following risk factors :

   - age,
   - hypertension,
   - diabete,
   - prior cerebrovascular accident or systemic embolism,
   - left atrium diameter greater that or equal to 50 mm by echocardiography,
   - left ventricular ejection fraction less than 40% by 2D-echocardiography.

8. Use according to any one of the preceding claims, **characterized in that** patients with permanent atrial fibrillation have additional risk factors corresponding to at least one of the following diseases:

   - hypertension,
   - structural heart disease,
   - tachycardia,
   - coronary heart disease,
   - non-rheumatic valvular heart disease,
   - ischemic dilated cardiomyopathy,
   - ablation for AF/AFL,
   - supra-ventricular tachycardia other than AF/AFL,
   - history of cardiac valve surgery,
   - non-ischemic dilated cardiomyopathy,
   - hypertrophic cardiomyopathy,
   - rheumatic valvular heart disease,
   - sustained ventricular tachycardia,
   - congenital heart disease,
   - ablation for other reason than AF/AFL,
   - ventricular fibrillation,

   and/or at least a cardiac device chosen among:

   - a pacemaker,
   - an implanted cardioverter defibrillator.

9. Use according to one of the preceding claims, **characterized in that**, for oral administration, dronedarone daily dose may reach 800 mg.

10. Method of prevention of cardiovascular events which comprises the administration to a patient with permanent atrial fibrillation of an effective dose of at least dronedarone or one of its pharmaceutically acceptable salts.

**EUROPEAN SEARCH REPORT**

Application Number

EP 10 30 5514

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/144550 A2 (SANOFI AVENTIS [FR]; GAUDIN CHRISTOPHE [FR]; HAMDANI NACERA [FR]; RADZ) 3 December 2009 (2009-12-03) <br> * claims 1, 6-10,18, 22-24, 47, 53-55, 61, 65-67 * <br> * example III.13 * <br> * page 3, line 36 - page 4, line 5 * <br> * page 20, lines 27-29 * <br> ----- | 1-10 | INV. <br> A61K31/343 <br> A61P9/00 |
| X | WO 2009/150535 A1 (SANOFI AVENTIS [FR]; GAUDIN CHRISTOPHE [FR]; HAMDANI NACERA [FR]; RADZ) 17 December 2009 (2009-12-17) <br> * claims 1-5 * <br> * page 9, line 34 - page 10, line 33 * <br> * page 2, lines 15-18 * <br> ----- | 1-10 | |
| X | Sanofi-aventis: "Sanofi-aventis commences multinational Phase IIIb PALLAS trial of Multaq in permanent AF patients" <br><br> 12 May 2010 (2010-05-12), XP002603798 <br> Retrieved from the Internet: <br> URL:http://www.news-medical.net/news/20100 512/Sanofi-aventis-commences-multinational -Phase-IIIb-PALLAS-trial-of-Multaq-in-perm anent-AF-patients.aspx <br> [retrieved on 2010-10-05] <br> * the whole document * <br> ----- | 1-10 | TECHNICAL FIELDS SEARCHED (IPC) <br><br> A61K |
| X | WO 2010/015939 A1 (SANOFI AVENTIS [FR]; RADZIK DAVIDE [FR]; VAN EICKELS MARTIN [DE]) 11 February 2010 (2010-02-11) <br> * claims 1-10 * <br> * page 12 - page 17 * <br> * page 6 - page 7 * <br> ----- <br><br> -/-- | 1-10 | |

The present search report has been drawn up for all claims

| Place of search | Date of completion of the search | Examiner |
|---|---|---|
| Munich | 8 October 2010 | Renard, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...............................................................................
& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 10 30 5514

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CRIJNS H J G M ET AL: "Effects of dronedarone on clinical outcomes in patients with atrial fibrillation and coronary heart disease: Insights from the ATHENA study" EUROPEAN HEART JOURNAL, OXFORD UNIVERSITY PRESS, GB, FR, vol. 30, no. SUPPL, 1 January 2009 (2009-01-01), page 450, XP002555809 ISSN: 0195-668X * abstract * ----- | 1-10 | |
| X | CONNOLLY S J: "Effect of Dronedarone on Stroke and Other Cardiovascular Outcomes" INTERNET CITATION 1 January 2008 (2008-01-01), XP009125920 Retrieved from the Internet: URL:http://resources.escardio.org/Webcast/ ESC-2008/4482/ [retrieved on 2009-11-27] * the whole document * ----- | 1-10 | |

TECHNICAL FIELDS SEARCHED (IPC)

The present search report has been drawn up for all claims

| Place of search | Date of completion of the search | Examiner |
|---|---|---|
| Munich | 8 October 2010 | Renard, Delphine |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 10 30 5514

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2010

| Patent document cited in search report | | Publication date | Patent family member(s) | | Publication date |
|---|---|---|---|---|---|
| WO 2009144550 | A2 | 03-12-2009 | PE | 18092009 A1 | 03-12-2009 |
| | | | US | 2010048694 A1 | 25-02-2010 |
| WO 2009150535 | A1 | 17-12-2009 | AR | 072069 A1 | 04-08-2010 |
| | | | EP | 2133074 A1 | 16-12-2009 |
| WO 2010015939 | A1 | 11-02-2010 | EP | 2153830 A1 | 17-02-2010 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0471609 B1

**Non-patent literature cited in the description**

- *Cochrane Collaboration, The Cochrane Library,* 2008, 2
- **D.G. WYSE.** *The New England Journal of Medecine,* 2002, vol. 347, 1825-1833
- **D. ROY.** *The New England Journal of Medecine,* 2008, vol. 358, 2667-2677
- **R.L. SACCO et al.** *Stroke,* 2006, vol. 37, 577-617